**Europäisches Patentamt**

**European Patent Office**

**Office européen des brevets**

(19)

(11) Veröffentlichungsnummer : **0 084 836**
**B1**

(12)

# EUROPÄISCHE PATENTSCHRIFT

(45) Veröffentlichungstag der Patentschrift :
03.12.86

(51) Int. Cl.⁴ : **C 07 C125/08**

(21) Anmeldenummer : **83100344.7**

(22) Anmeldetag : **17.01.83**

(54) Verfahren zur Herstellung der Alkalimetallsalze von Acylcyanamiden.

(30) Priorität : 25.01.82 DE 3202213

(43) Veröffentlichungstag der Anmeldung :
03.08.83 Patentblatt 83/31

(45) Bekanntmachung des Hinweises auf die Patenterteilung : 03.12.86 Patentblatt 86/49

(84) Benannte Vertragsstaaten :
AT BE CH DE FR GB IT LI NL SE

(56) Entgegenhaltungen :
DE-A- 2 757 586
DE-C-  708 428

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Hase, Christian, Dr.**
**Millrather Weg 29**
**D-4006 Erkrath 1 (DE)**
Erfinder : **Baumann, Horst, Dr.**
**Vogelwarte 17**
**D-5653 Leichlingen (DE)**
Erfinder : **Carduck, Franz-Josef, Dr.**
**Landstrasse 18**
**D-5657 Haan (DE)**
Erfinder : **Pawelczyk, Hubert, Dr.**
**Alt Eller 23**
**D-4000 Düsseldorf 1 (DE)**

## Beschreibung

Die Erfindung betrifft ein im technischen Maßstab anwendbares, auch in kontinuierlicher Arbeitsweise durchführbares Verfahren zur Herstellung der Alkalimetallsalze von Acylcyanamiden, insbesondere aliphatischer Carbonsäuren, unter Verwendung der Carbonsäureester von niederen Alkoholen und Monoalkalicyanamiden als Reaktionspartner.

Acylcyanamide in Form ihrer Alkalisalze sind seit langer Zeit bekannte Verbindungen. So wurde bereits im Jahre 1878 das Natriumsalz des Acetylcyanamids beschrieben (J. prakt. Chemie. 17 (1878), Seite 9 bis 11). Aus der britischen Patentschrift 428 091 des Jahres 1935 und ebenfalls aus der im Jahre 1941 bekanntgemachten deutschen Patentschrift 708 428 sind die Natriumsalze der Acylcyanamide von langkettigen Fettsäuren, Harzsäuren oder Naphthensäuren und deren Verwendung als seifenähnliche Tenside für Wasch- und Netzmittel bekannt. Zur Herstellung der sowohl kurzkettigen als auch langkettigen Acylcyanamide wurden die entsprechenden Säurechloride oder Säureanhydride zunächst mit einem Überschuß an Cyanamid zum Acylcyanamid umgesetzt, das dann in einem weiteren Schritt in das Alkalisalz übergeführt wurde. Aus den deutschen Offenlegungsschriften 20 22 491 und 20 22 492 ist ein Verfahren zur Herstellung von bifunktionellen Acylcyanamiden, beispielsweise Adipinsäuredicyanamid, durch Umsetzen der Dicarbonsäurechloride bzw. Dicarbonsäureester mit Cyanamid in alkoholischer Lösung in Gegenwart von alkalisch wirkenden Mitteln und durch Erhitzen am Rückfluß bekannt. In ähnlicher Weise wird in der deutschen Offenlegungsschrift 27 57 586 ein Verfahren zur Herstellung von Natriumformylcyanamid durch Umsetzen von Ameisensäureethylester mit Natriumcyanamid in alkoholischer Lösung durch Erhitzen unter Rückfluß beschrieben. Schließlich sollen nach der europäischen Offenlegungsschrift 8 475 substituierte Cyanoamine, unter deren allgemeine Formel auch Acylcyanamide von $C_1$-$C_{20}$-Carbonsäuren fallen, durch Umsetzen der Amine bzw. Amide mit Bromcyan in Benzol herstellbar sein ; das entsprechende Natriumsalz soll durch Lösen in konzentrierter Natronlauge, Versetzen mit Ammoniumcarbonat und Extrahieren mit Isopropylalkohol erhalten werden können.

Obwohl es sich also bei den Acylcyanamiden der langkettigen Fettsäuren um seifenähnliche Tenside handelt, die seit fast 50 Jahren bekannt sind, hat dieser Verbindungstyp als Waschmittelrohstoff bisher keine technische Beachtung gefunden. Diese Tatsache hat nicht zuletzt ihren Grund in den für die Produktion in technischem Maßstab ungeeigneten Herstellungsbedingungen nach den bekannten Verfahren. Diese Verfahren waren im Falle der Fettsäurechloride oder Fettsäureanhydride als Ausgangsstoffe schwierig und langwierig, weil zunächst die freien Acylcyanamide erhalten werden und die Herstellung der Alkalisalze einen weiteren Schritt erfordert.

Es wurde nun gefunden, daß sich die Alkalimetallsalze der Acylcyanamide, insbesondere der Acylcyanamide von aliphatischen Carbonsäuren, durch Umsetzung der entsprechenden Carbonsäureester mit Monoalkalicyanamid nach einem Verfahren herstellen lassen, das sich auch mit Vorteil in den technischen Maßstab übertragen läßt. Das neue Herstellungsverfahren ist dadurch gekennzeichnet, daß man den Carbonsäureester eines niederen Alkohols mit einem festen, vorzugsweise wasserfreien Monoalkalisalz des Cyanamids in äquimolaren Mengen vermischt und diese Mischung auf Temperaturen von 100-300 °C erhitzt.

Das erfindungsgemäße Verfahren benötigt im Prinzip kein Lösungsmittel ; überraschenderweise wurde vielmehr festgestellt, daß die Reaktion zwischen Ester und dem Alkalisalz des Cyanamids auch ohne Anwesenheit eines alkoholischen Lösungsmittels abläuft. Allerdings sind bei dem erfindungsgemäßen Verfahren höhere Initialtemperaturen als die Rückflußtemperaturen der niederen Alkohole, d. h. Temperaturen oberhalb 100 °C, erforderlich. Der bei der Bildung des Acylcyanamids freigesetzte niedere Alkohol sammelt sich im Reaktionsmilieu an und kann daraus unter den geeigneten Druckbedingungen durch Destillation entfernt werden. Bevorzugt wird man die Ester der niederen Alkohole Methanol, Ethanol oder Isopropylalkohol einsetzen und die Reaktion unter Normaldruck durchführen, so daß der Alkohol in dem Maße, wie er entsteht, abdestilliert und die abdestillierte Alkoholmenge ein Maß für den Fortgang der Reaktion darstellt. Das Reaktionsende ist sinngemäß dann erreicht, wenn kein weiterer Alkohol entsteht und abdestilliert. Bei dem erfindungsgemäßen Verfahren kann der freigesetzte niedrigsiedende Alkohol an sich auch im Reaktionsgemisch verbleiben, wenn man die Umsetzung in einem Druckgefäß durchführt. Der dabei auftretende Vorteil der leichteren Rührbarkeit des Reaktionsgemisches wird aber durch den Nachteil, daß in einem Druckgefäß, also einer aufwendigen Apparatur, gearbeitet werden muß, mehr als aufgewogen. Verwendet man andererseits bei dem erfindungsgemäßen Verfahren den Ester eines Alkohols, der bei Normaldruck oberhalb der gewählten Reaktionstemperatur siedet, dann kann die Umsetzung auch unter vermindertem Druck, bei dem der freigesetzte Alkohol abdestilliert, durchgeführt werden.

Vorzugsweise wird das erfindungsgemäße Verfahren aus Gründen der technischen Einfachheit bei Normaldruck und ohne Zusatz eines niedrigsiedenden Alkohols als Lösungsmittel durchgeführt. Die Verfahrensprodukte werden unter diesen Bedingungen unmittelbar nach dem Abkühlen in fester Form erhalten, wobei es ohne weiteres möglich ist, das zunächst in der Wärme als viskose Schmelze anfallende Verfahrensprodukt beim Abkühlen in Prills, Nadeln, Schuppen, Pulver oder sonstige, für die Weiterverarbeitung zweckmäßige feste Formen überzuführen. Durch die gegenüber den bekannten Verfahren wesentlich höheren Reaktionstemperaturen, bei denen der sich bildende Alkohol ständig abdestilliert, wird die Reaktionszeit stark verkürzt. Im übrigen erlaubt es das Arbeiten ohne Lösungsmittel auch, das erfindungsgemäße Verfahren als kontinuierliches Verfahren durchzuführen.

Unter Acylcyanamiden werden generell die Carbonsäurecyanamide mit an sich beliebigen Gruppierungen im Acylrest verstanden. Demnach können Acylcyanamide aliphatischer, aromatischer, alkylaromatischer und heterocyclischer Carbonsäuren in Form ihrer Alkalisalze nach dem erfindungsgemäßen Verfahren hergestellt werden. Die Acylreste können auch beliebig substituiert sein bzw. heteroatomare Brückenglieder, wie z. B. eine Oxy- oder Iminogruppe aufweisen. Die Struktur der möglichen Acylreste wird lediglich durch die an sich selbstverständliche Forderung limitiert, daß die Acylreste bzw. ihre Substituenten und Brückenglieder unter den Reaktionsbedingungen inert sind. Der Begriff Acylcyanamide umfaßt auch die Derivate von Di- oder Tricarbonsäuren bzw. allgemein von Polycarbonsäuren.

Das erfindungsgemäße Verfahren betrifft in erster Linie jedoch die Herstellung der Alkalisalze von Acylcyanamiden von aliphatischen Carbonsäuren, vorzugsweise von Monocarbonsäuren, und hier ganz besonders die Herstellung der Acylcyanamid-Alkalisalze von langkettigen aliphatischen Carbonsäuren mit 6 bis 30 Kohlenstoffatomen im Acylrest. Die Herstellung der Alkalisalze von Acylcyanamiden der $C_6$-$C_{30}$-Fettsäuren, insbesondere der $C_{10}$-$C_{24}$-Fettsäuren, sind ein ganz besonderes Ziel der Erfindung. Davon sind die Fettsäurecyanamid-Alkalisalze der $C_{12}$-$C_{18}$-Fettsäuren in erster Linie als Tenside für die Verwendung in Wasch- und Reinigungsmitteln geeignet. Fettsäurecyanamid-Alkalisalze können nunmehr nach dem erfindungsgemäßen Verfahren in grosser Reinheit und in großen Mengen hergestellt werden. Die Alkalisalze der Fettsäurecyanamide werden durch die Erfindung zu einem Tensidrohstoff auf Fettbasis von technischer Bedeutung. Auch wegen der gegenüber den üblichen Seifen geringeren Hydrolyseanfälligkeit sind die erfindungsgemäß hergestellten Alkalisalze der Fettsäurecyanamide interessante Wirkstoffe für die Formulierung neuer Wasch- und Reinigungsmittelrezepturen.

Bei dem als Ausgangsstoff benutzten Carbonsäureester eines niederen Alkohols handelt es sich insbesondere um den Ester eines einwertigen niederen Alkohols mit 1 bis 5 C-Atomen ; d. h. es werden die Methyl-, Ethyl-, Propyl- ester, ferner die Butyl- und Pentylester als Ausgangsstoffe eingesetzt. Prinzipiell können aber auch die Carbonsäureester von niederen zwei- und dreiwertigen Alkoholen eingesetzt werden. Unter bestimmten Voraussetzungen sind als Ausgangsstoffe auch die Carbonsäureester des Glycerins, also insbesondere die als Triglyceride vorliegenden natürlichen Fette geeignet. Es können als Carbonsäureester dann die Fettsäuretriglyceride eingesetzt werden, wenn das bei dem Verfahren entstehende Glycerin bei der späteren technischen Verwendung des Verfahrensprodukts als Komponente nicht stört oder seine Anwesenheit sogar erwünscht ist. Beim Einsatz der Fettsäuretriglyceride als Ausgangsstoffe ist es deshalb auch nicht notwendig, die Reaktionsbedingungen so zu wählen, daß das freigewordene Glycerin abdestilliert, obwohl die Abdestillation durch die Wahl der passenden Reaktionstemperatur- und Druckbereiche im Prinzip möglich ist.

Die Wahl des Carbonsäureesters wird jedoch im allgemeinen durch Kriterien, wie leichte technische Verfügbarkeit des Esters und einfache Durchführung des Verfahrens bestimmt. Die Fettsäuremethylester sind deshalb für technische Verfahrensansätze die Carbonsäureester der Wahl.

Das feste Cyanamid wird als Monoalkalisalz des Lithiums, Natriums oder Kaliums bei dem erfindungsgemäßen Verfahren als zweite Reaktionskomponente eingesetzt. Vorzugsweise wird hauptsächlich aus preislichen Gründen das Mononatriumsalz des Cyanamids eingesetzt. Bei dem Verfahren wird dem trockenen, d. h. wasserfreien Alkalisalz des Cyanamids dann der Vorzug gegeben, wenn man auf die Abwesenheit von üblicher Seife als Nebenprodukt Wert legt. In vielen Fällen kann jedoch für die spätere Weiterverwendung des Verfahrensprodukts die Anwesenheit der üblichen Alkaliseife als Nebenprodukt unbedenklich oder sogar erwünscht sein.

Die Reaktionstemperatur ist bei dem erfindungsgemäßen Verfahren in weiten Grenzen varrierbar. Der untere Grenzwert für den Reaktionstemperaturbereich ist im wesentlichen von der Lage der Initialtemperatur, bei der die beiden innig vermischten Reaktionskomponenten miteinander zu reagieren beginnen, abhängig, während die Obergrenze der Reaktionstemperatur praktisch durch die Berücksichtigung und Vermeidung von unerwünschten Nebenreaktionen, insbesondere Verfärbungen, festgelegt ist. Für die technische Produktion hat sich eine Reaktionstemperatur im Bereich von 100 bis 250 °C als zweckmäßig und bevorzugt erwiesen. Wählt man für das Verfahren Reaktionstemperaturen im Bereich von 250 °C oder darüber, so kann man durch das Einhalten der unter diesen Bedingungen nur kurzen Verfahrensdauer das Problem der thermischen Zersetzung vermeiden.

Im allgemeinen kann der Fortgang des Verfahrens an der Alkoholbildung gemessen werden, bzw. man bestimmt die Menge des noch verbliebenen Esters, z. B. mit Hilfe der Gaschromatographie oder als wasserunlöslichen Anteil. Gut geeignet zur Bestimmung der Reaktionspartner ist auch die Infrarot-Spektroskopie, da alle Reaktionspartner charakteristische starke Absorptionen bei verschiedenen Wellenlängen aufweisen, z. B. :

| | |
|---|---|
| NaHNCN : | 2 120 und 2 170 cm$^{-1}$ ; |
| Fettsäureester : | um 1 750 cm$^{-1}$ ; |
| Stearinsäurecyanamid-Li : | 2 180, 1 600 und 1 390 cm$^{-1}$ ; |
| Stearinsäurecyanamid-Na : | 2 160, 1 515 und 1 390 cm$^{-1}$ ; |
| Stearinsäurecyanamid-K : | 2 160, 1 560 und 1 380 cm$^{-1}$ . |

Die Alkalisalze der Fettsäurecyanamide sind bei Raumtemperatur feste farblose bis leicht gelblich gefärbte Stoffe von spröder bis wachsartiger Beschaffenheit. Sie erweichen bei höheren Temperaturen und schmelzen oberhalb 100 bis 150 °C je nach ihrer Zusammensetzung zu zähen Flüssigkeiten. Die Schmelzpunkte sind nicht charakteristisch. Bei der Verwendung der technisch besonders interessanten Fettsäureester aus Fettsäuren natürlicher Herkunft handelt es sich im allgemeinen um Gemische, d. h. um Verbindungen mit unterschiedlich langen Fettsäureresten, so daß hier ein scharfer Schmelzpunkt als charakteristisches Substanzmerkmal nicht erwartet werden kann.

Die Auswahl der Vorrichtungen, in denen das erfindungsgemäße Verfahren durchgeführt werden kann, richtet sich nach den Ansatzmengen und nach den rheologischen Eigenschaften bei der vorgesehenen Reaktionstemperatur. So kommen z. B. gewöhnliche beheizbare Rührkessel, Kneter aber auch Rohrreaktoren mit Einrichtungen zum Wiedergewinnen des freigesetzten Alkohols in Betracht. Nach beendeter Reaktion können die Verfahrensprodukte als Schmelzen, z. B. durch beheizte Leitungen, direkt der Weiterverwendung zugeführt werden. Nach einer anderen Aufarbeitungsvariante können die Schmelzen aber auch durch Verprillen, Vernadeln oder Verschuppen in eine für die Lagerung oder Weiterverwendung geeignete feste Form gebracht werden. Auch die Herstellung eines wäßrigen Konzentrats durch Auflösen in Wasser ist möglich, doch bedeutet gerade die Tatsache, daß die Alkalisalze der Fettsäurecyanamide unmittelbar in trockener Form enthalten werden können, bei vielen Anwendungen einen Vorteil. Bekanntlich werden die wichtigsten technischen Tenside vom Typ der Sulfonate und Sulfate als wäßrige pastenförmige Konzentrate bei der großtechnischen Weiterverwendung eingesetzt. Der Wassergehalt dieser Pasten wird jedoch häufig als Nachteil angesehen, beispielsweise bei der Herstellung von Waschmittelpulvern durch Heißsprühtrocknung eines wäßrigen Ansatzes (Slurry) in Sprühtürmen. Bei diesem großtechnisch bedeutenden Verfahren zur Herstellung von pulverförmigen Waschmitteln ist es von Vorteil, wenn die Wirkstoffe möglichst wasserarm sind, um den Wassergehalt im Slurry und damit auch die bei der anschließenden Zerstäubungstrocknung erforderlichen Energiemengen zur Entfernung des Wassers niedrig halten zu können.

Ein weiterer Aspekt der Erfindung bezieht sich auf die Bereitstellung der Alkalisalze der Acylcyanamide in einer Form, welche die meist hohe Viskosität der reinen Verbindungen in der Schmelze nicht aufweist und die deshalb besser pump- und rührbar ist. Diese weitere Ausführungsform der Erfindung ist durch den Zusatz einer hochsiedenden wasserlöslichen, unter den Reaktionsbedingungen inerten Substanz als Flußmittel zu den Reaktionskomponenten gekennzeichnet. Als derartige Flußmittel eignen sich in erster Linie Substanzen, deren Anwesenheit bei der späteren Weiterverwendung des Verfahrensprodukts nicht stört ; insbesondere eignen sich solche Substanzen als Zusätze, deren Anwesenheit bei der Weiterverwendung des Verfahrensprodukts ausdrücklich erwünscht ist. Im Falle der Weiterverwendung der Verfahrensprodukte als Tenside für die Herstellung von Wasch- und Reinigungsmitteln handelt es sich bei den geeigneten Flußmittel-Zusätzen insbesondere um Polymerisationsprodukte mit Ethylenoxid und/oder Propylenoxid, wie z. B. um Polyethylenglykole, Blockpolymerisate aus Ethylenoxid und Propylenoxid, um Ethylenoxid- und/oder Propylenoxid-Addukte an langkettige Alkohole, Alkylphenole oder Fettsäureamide. Zu dieser Gruppe von Verbindungen gehören auch die nichtionischen Tenside, die häufig Bestandteile von Wasch- und Reinigungsmittelrezepturen sind. Der Zusatz eines Flußmittels bewirkt im allgemeinen auch eine Senkung der Initialtemperatur. Im weitesten Sinne ist das beim Einsatz von Fettsäuretriglyceriden als Ausgangsstoff *in situ* entstehende Glycerin ebenfalls als Flußmittel anzusehen. In diesem Fall werden die Verfahrensbedingungen bezüglich Temperatur und Druck vorzugsweise so gewählt, daß das freigesetzte Glycerin im Reaktionsmilieu verbleibt. Als Flußmittel eignen sich ebenfalls die Alkalisalze der Fettsäuren, d. h. die üblichen Seifen. Die Seife braucht als Flußmittel ebenfalls nicht separat hinzugesetzt zu werden ; vielmehr ist es in diesem Fall möglich und auch zweckmäßig, ein nicht vollständig getrocknetes, d. h. noch wasserhaltiges Alkalicyanamid als Reaktionspartner einzusetzen, so daß die Seife als Flußmittel bei der Reaktion *in situ* gebildet wird. Zum im wesentlichen gleichen Ergebnis kommt man auch, wenn man trockenes Alkalicyanamid zusammen mit Alkalihydroxid einsetzt.

## Beispiele

Die Erfindung wird durch die folgenden Beispiele erläutert.

## Beispiel 1

Vom Methylester der Kokosvorlauffettsäuren (Verseifungszahl 332) mit im wesentlichen $C_6$-$C_{12}$-Fettsäuren wurden 253 g (1,5 Mol) in einem 1 1-Dreihalskolben mit Rührer, Innenthermometer und aufgesetzter Destillationsbrücke mit 96 g (1,5 Mol) trockenem Mononatriumcyanamid gut verrührt und die Mischung auf 155° erhitzt. Die Hauptmenge des entstehenden Methanols destillierte im Verlauf von 2 Stunden ab, wobei der Kolbeninhalt verdickte aber rührbar blieb. Der Kolbeninhalt wurde ausgegossen und erstarrte zu einem spröden weißlichen Festprodukt, das klar in Wasser löslich war. Das Infrarot-Spektrum des Reaktionsprodukts besaß charakteristische Absorptionen bei 2 160, 1 515 und 1 390 $cm^{-1}$, wodurch das Produkt als Carbonsäurecyanamid-Natriumsalz ausgewiesen wurde.

## Beispiel 2

In der in Beispiel 1 beschriebenen Apparatur wurden auf gleiche Weise 270,5 g (1 Mol) technisches Isopropylmyristat mit 64 g (1 Mol) Mononatriumcyanamid während 2,5 Stunden auf 170 °C erhitzt. Der bei der Reaktion entstandene Isopropylalkohol war bereits nach 2 Stunden nahezu vollständig abdestilliert. Das Reaktionsprodukt wurde als hochviskose Paste, die nicht mehr aus dem Kolben ausgießbar war, erhalten. Das Produkt erstarrte beim Abkühlen zu einem spröden weißlichen Feststoff, der klar in Wasser löslich war. Die Ausbeute war quantitativ.

## Beispiel 3

In einem 1 1-Dreihalskolben mit Rührer und Thermometer wurden 289,2 g (0,33 Mol) eines technischen Triglyceridgemisches von Palmitin- und Stearinsäure (gehärteter Talg) (Verseifungszahl 194) mit 64 g (1 Mol) Mononatriumcyanamid auf 160 °C erhitzt. Bereits nach 15 Minuten verdickte der Kolbeninhalt ; er blieb jedoch gut rührbar. Das Reaktionsgemisch wurde nach 30 Minuten bei 160° ausgegossen und erstarrte beim Abkühlen zu einem wachsartigen Feststoff, der sich klar in Wasser löste. Ausbeute 345 g (97 %). Das Infrarotspektrum des Produkts zeigte neben den charakteristischen Acylcyanamidbanden die des Glycerins.

## Beispiel 4

In einem 500 ml Dreihalskolben mit Rührer, Thermometer und aufgesetzter Destillationsbrücke wurden 100 g (0,42 Mol) eines Kokosfettsäuremethylesters (Verseifungszahl 235) (Kettenlänge $C_{12}$-$C_{18}$) und 27 g (0,42 Mol) trockenes Mononatriumcyanamid vermischt und mit einem auf 260 °C vorgeheiztem Ölbad schnell auf 240° Innentemperatur erhitzt. Nach 15 Minuten begann die Methanolentwicklung, die bereits nach weiteren 10 Minuten beendet war. Dabei wurde die Reaktionsmischung zähflüssig. Nach insgesamt 30 Minuten wurde von einer Probe das Infrarotspektrum aufgenommen und die Abwesenheit der Esterabsorptionsbande festgestellt. Das zähflüssige Produkt wurde ausgegossen und erstarrte zu einem gelblichen in Wasser leicht löslichen Feststoff. Das Produkt zeigte das charakteristische IR-Spektrum der Fettsäurecyanamid-Natriumsalze. Ausbeute 256 g (95 %).

## Beispiel 5

In der Apparatur des Beispiels 4 wurden 143,7 g (0,5 Mol) des Methylesters der hydrierten Talgfettsäuren (Verseifungszahl 195) und 40 g (0,5 Mol) Monokaliumcyanamid vermischt und 2 Stunden lang auf 160 °C-170 °C erhitzt. Dann wurde das zähflüssige Reaktionsprodukt ausgegossen ; es erstarrte beim Abkühlen zu einem gelblichen, wasserlöslichen Feststoff. Durch sein Infrarotspektrum wurde das Produkt als Acylcyanamid-Kaliumsalz identifiziert. Ausbeute 154 g (92 %).

## Beispiel 6

In der in Beispiel 1 beschriebenen Apparatur wurden 230,4 g des Methylesters der Kokosfettsäuren (Verseifungszahl 243,5) mit 64 g (1 Mol) Mononatriumcyanamid und 50 g des Anlagerungsprodukts von 10 Mol Ethylenoxid an Talgalkohol (Handelsname Eumulgin 010, eingetragenes Warenzeichen) vermischt und auf 160° erhitzt. Nach 3 Stunden war die Reaktion beendet, nachdem praktisch die berechneten Mengen Methanol abdestilliert waren. Nach einer Gesamtreaktionszeit von 5 Stunden wurde der dünnflüssige und einwandfrei rührbare Kolbeninhalt ausgegossen. Nach dem Abkühlen wurde das Reaktionsprodukt als wachsartige, weißgelbliche gut wasserlösliche Masse erhalten. Das Produkt zeigte neben den charakteristischen Banden der Fettsäurecyanamid-Natriumsalze die typischen Banden aliphatischer Ether. Ausbeute 289 g (93 %).

## Beispiel 7

In einem Ansatz, der im übrigen dem in Beispiel 1 entsprach, wurden anstelle von 1,5 Mol Mononatriumcyanamid nur 1,3 Mol dieses Salzes zusammen mit 0,2 Mol NaOH eingesetzt. Während der Reaktion bildete sich neben dem Natriumacylcyanamid die Natriumseife. Die Reaktionsmischung war leichter rührbar und erstarrte nach dem Ausgießen zu einem hellfarbenen, wachsartigen Festprodukt. Sein Infrarotspektrum zeigte neben den Banden des Acylcyanamidsalzes die Carbonylbande der Seife bei 1 560 cm$^{-1}$.

## Beispiel 8

In Analogie zu Beispiel 2 wurden in 3 verschiedenen Ansätzen jeweils 298,5 g (1 Mol) Stearinsäuremethylester mit jeweils 1 Mol Monolithiumcyanamid (48 g), bzw. 1 Mol Mononatriumcyanamid (64 g), bzw. 1 Mol Monokaliumcyanamid (80 g) bei 150-170 °C umgesetzt. Reaktionsdauer 2-3 Stunden. Die Produkte Stearinsäurecyanamidlithiumsalz bzw. Stearinsäurecyanamidnatriumsalz bzw. Stearinsäurecyanamidkaliumsalz fielen mit quantitativer Ausbeute als schwach gelbliche Feststoffe an.

Beispiel 9

Dieses Beispiel beschreibt eine kontinuierliche Arbeitsweise des erfindungsgemäßen Verfahrens. Eine auf 60 °C erwärmte Suspension von 6,80 kg (106,3 Mol) trockenem Mononatriumcyanamid in 25,0 kg (106,3 Mol) eines Methylesters der Kokosfettsäuren ($C_{12}$-$C_{18}$) mit der Verseifungszahl 238 wurde kontinuierlich einem Schneckenreaktor zugeführt, der auf 240 °C geheizt wurde. Die Zulaufgeschwindigkeit betrug 0,6 Liter/Minute, so daß sich bei dem gegebenen effektiven Reaktorvolumen von zirka 11 Liter eine mittlere Verweilzeit von zirka 20 Minuten einstellte. Das über die Brüdenausgänge entweichende Methanol wurde in einem absteigenden Glaskühler kondensiert, während das entstandene Fettsäurecyanamidnatriumsalz mit Hilfe eines angeschlossenen Extruders auf eine Schuppenwalze gefördert wurde. Das Produkt erstarrte auf der mit Leitungswasser gekühlten Walze und wurde in Form schwach gelb gefärbter, vollständig wasserlöslicher Schuppen gewonnen. Das Infrarotspektrum des Produkts zeigte bei 2 160, 1 515 und 1 390 cm$^{-1}$ die für Fettsäurecyanamidnatriumsalze charakteristischen Banden. Die Ausbeute betrug 27,9 kg (98 %) ; daneben wurden 3,1 kg Methanol (91 %) aufgefangen.

**Patentansprüche**

1. Verfahren zur Herstellung von Alkalimetallsalzen der Acylcyanamide von insbesondere aliphatischen Carbonsäuren durch Umsetzung der Carbonsäureester mit Monoalkalicyanamiden, dadurch gekennzeichnet, daß man einen Carbonsäureester eines niederen Alkohols mit einem festen, vorzugsweise wasserfreien Monoalkalisalz des Cyanamids in äquimolaren Mengen ohne Zusatz eines niedrigsiedenden Alkohols als Lösungsmittel vermischt und diese Mischung auf Temperaturen von 100 bis 300 °C erhitzt.

2. Verfahren nach Anspruch 1, dadurch gekennzeichnet, daß man es unter normalem Druck durchführt.

3. Verfahren nach Anspruch 1 und 2, dadurch gekennzeichnet, daß man eine Reaktionstemperatur wählt, bei der der freigesetzte Alkohol abdestilliert.

4. Verfahren nach Ansprüch 1 bis 4, dadurch gekennzeichnet, daß die Reaktionstemperatur im Bereich von 100-250 °C liegt.

5. Verfahren nach Anspruch 1 bis 5, dadurch gekennzeichnet, daß man als Carbonsäureester den Ester einer aliphatischen Carbonsäure mit 6-30 Kohlenstoffatomen im Carbonsäurerest einsetzt.

6. Verfahren nach Anspruch 1 bis 6, dadurch gekennzeichnet, daß man als Carbonsäureester einen Ester der $C_6$-$C_{30}$-Fettsäuren, insbesondere der $C_{10}$-$C_{24}$-Fettsäuren einsetzt.

7. Verfahren nach Anspruch 1 bis 7, dadurch gekennzeichnet, daß man als Carbonsäureester einen Ester einer $C_{12}$-$C_{18}$-Fettsäure, insbesondere den Methylester einsetzt.

8. Verfahren nach Anspruch 1 bis 8, dadurch gekennzeichnet, daß man als Monoalkalicyanamid das Mononatriumsalz des Cyanamids, vorzugsweise in wasserfreier Form, einsetzt.

9. Verfahren nach Anspruch 1 bis 9, dadurch gekennzeichnet, daß man den Reaktionskomponenten eine hochsiedende wasserlösliche, unter den Reaktionsbedingungen inerte flüssige oder pastöse Substanz als Flußmittel zusetzt.

10. Verfahren nach Anspruch 10, dadurch gekennzeichnet, daß man als Flußmittel ein Polymerisationsprodukt mit Ethylenoxid und/oder Propylenoxid, insbesondere vom Typ der nichtionischen Tenside, einsetzt.

11. Verfahren nach Anspruch 1 bis 9, dadurch gekennzeichnet, daß man als Carbonsäureester ein Fettsäuretriglycerid als Reaktionskomponente verwendet.

12. Verfahren nach Anspruch 1 bis 9, dadurch gekennzeichnet, daß man wasserhaltiges Alkalicyanamid oder ein Gemisch aus trockenem Alkalicyanamid und Alkalihydroxid als Reaktionspartner einsetzt.

**Claims**

1. A process for the production of alkali metal salts of acylcyanamides of, in particular, aliphatic carboxylic acids by reaction of the carboxylic acid esters with monoalkali cyanamides, characterized in that a carboxylic acid ester of a lower alcohol is mixed with a solid, preferably anhydrous monoalkali salt of cyanamide in equimolar quantities in the absence of a low-boiling alcohol as solvent and the resulting mixture is heated to temperatures of from 100 to 300 °C.

2. A process as claimed in Claim 1, characterized in that it is carried out under normal pressure.

3. A process as claimed in Claims 1 and 2, characterized in that the reaction is carried out at a temperature at which the alcohol released distills off.

4. A process as claimed in Claims 1 to 4, characterized in that the reaction temperature is in the range of from 100 to 250 °C.

5. A process as claimed in Claims 1 to 5, characterized in that the ester of an aliphatic carboxylic acid containing from 6 to 30 carbon atoms in the carboxylic acid residue is used as the carboxylic acid ester.

6. A process as claimed in Claims 1 to 6, characterized in that an ester of $C_6$-$C_{30}$ fatty acids and, more especially, $C_{10}$-$C_{24}$ fatty acids is used as the carboxylic acid ester.

7. A process as claimed in Claims 1 to 7, characterized in that an ester of a $C_{12}$-$C_{18}$ fatty acids, more especially the methyl ester, is used as the carboxylic acid ester.

8. A process as claimed in Claims 1 to 8, characterized in that the monosodium salt of cyanamide, preferably in anhydrous form, is used as the monoalkali cyanamide.

9. A process as claimed in Claims 1 to 9, characterized in that a high-boiling, water-soluble liquid or pasty substance inert under the reaction conditions is added as flux to the reaction components.

10. A process as claimed in Claim 10, characterized in that a polymerization product with ethylene oxide and/or propylene oxide, particularly of the nonionic surfactant type, is used as flux.

11. A process as claimed in Claims 1 to 9, characterized in that a fatty acid triglyceride is used as the carboxylic acid ester reaction component.

12. A process as claimed in Claims 1 to 9, characterized in that aqueous alkali cyanamide or a mixture of dryalkali cyanamide and alkali hydroxide is used as reactant.


## Revendications

1. Procédé pour la fabrication de sels de métaux alcalins des acylcyanamides, en particulier d'acides carboxyliques aliphatiques, par réaction de l'ester des acides carboxyliques avec des cyanamides mono-alcalines, caractérisé en ce que l'on mélange un ester d'acide carboxylique d'un alcool inférieur avec un sel mono-alcalin solide, de préférence anhydre, de la cyanamide, en porportions équimoléculaires, sans addition d'un alcool à bas point d'ébullition comme solvant et chauffe ce mélange à des températures de 100 à 300 °C.

2. Procédé suivant la revendication 1, caractérisé en ce qu'il est effectué à la pression normale.

3. Procédé suivant les revendications 1 et 2, caractérisé en ce que l'on choisit une température à laquelle l'alcool libéré distille.

4. Procédé suivant les revendications 1 à 3, caractérisé en ce que la température de réaction est de l'ordre de 100 à 250 °C.

5. Procédé suivant les revendications 1 à 4, caractérisé en ce que l'on utilise comme ester d'acide carboxylique, l'ester d'un acide carboxylique aliphatique contenant 6 à 30 atomes de carbone dans le reste acide carboxylique.

6. Procédé suivant les revendications 1 à 5, caractérisé en ce que l'on utilise comme ester d'acide carboxylique, un ester d'acides gras en $C_6$ à $C_{30}$, en particulier d'acides gras en $C_{10}$ à $C_{24}$.

7. Procédé suivant les revendications 1 à 6, caractérisé en ce que l'on utilise comme ester d'acide carboxylique, un ester d'un acide gras en $C_{12}$ à $C_{18}$, en particulier l'ester méthylique.

8. Procédé suivant les revendications 1 à 7, caractérisé en ce que l'on utilise, comme cyanamide monoalcaline, le sel monosodique de la cyanamide, de préférence sous forme anhyde.

9. Procédé suivant les revendications 1 à 8, caractérisé en ce que l'on ajoute aux composants de la réaction, comme dilutif, une substance inerte dans les conditions de la réaction, liquide ou pâteuse, soluble à l'eau à haut point d'ébullition.

10. Procédé suivant la revendication 9, caractérisé en ce que l'on ajoute, comme dilutif, un produit de polymérisation avec de l'oxyde d'éthylène et/ou de l'oxyde de propylène, en particulier du type des agents tensio-actifs non-ioniques.

11. Procédé suivant les revendications 1 à 8, caractérisé en ce que l'on utilise, comme ester d'un acide carboxylique, un triglycéride d'acide gras, comme composant de la réaction.

12. Procédé suivant les revendications 1 à 8, caractérisé en ce que l'on utilise, comme participant à la réaction, une cyanamide alcaline contenant de l'eau, ou un mélange de cyanamide alcaline sèche et d'hydroxyde alcalin.